(19) **Europäisches Patentamt / European Patent Office / Office européen des brevets**

(11) **EP 0 788 335 B1**

(12) **EUROPEAN PATENT SPECIFICATION**

(45) Date of publication and mention
of the grant of the patent:
**03.10.2007 Bulletin 2007/40**

(51) Int Cl.:
**A61F 13/15** *(2006.01)*   **A61F 13/20** *(2006.01)*

(21) Application number: **95941446.7**

(22) Date of filing: **21.11.1995**

(86) International application number:
**PCT/US1995/015190**

(87) International publication number:
**WO 1996/016622 (06.06.1996 Gazette 1996/26)**

(54) **ABSORBENT ARTICLE**

ABSORBIERENDER ARTIKEL

ARTICLE ABSORBANT

(84) Designated Contracting States:
**AT BE CH DE DK ES FR GB GR IE IT LI LU NL PT SE**

(30) Priority: **01.12.1994 IT TO940982**

(43) Date of publication of application:
**13.08.1997 Bulletin 1997/33**

(73) Proprietor: **THE PROCTER & GAMBLE COMPANY**
**Cincinnati, Ohio 45202 (US)**

(72) Inventors:
• **D'ALESSIO, Nicola**
**I-65126 Pescara (IT)**

• **CARLUCCI, Giovanni**
**I-66100 Chieti (IT)**

(74) Representative: **Veronese, Pancrazio et al**
**Procter & Gamble Italia S.p.A.**
**Italian Research Center**
**Via Aterno 92/94**
**66020 Sambuceto di San Giovanni Teatino**
**(Chieti) (IT)**

(56) References cited:
**EP-A- 0 108 637**   **EP-A- 0 395 501**
**WO-A-93/15702**   **WO-A-94/06385**
**US-A- 5 437 653**

Printed by Jouve, 75001 PARIS (FR)

**Description**

This invention relates to an absorbent article. It is particularly concerned with an absorbent article, for example in the form of a pad, which can be used by women suffering from light and moderate incontinence. The invention will be so described below. However, the invention is of more general applicability in relation to the absorption of body fluids, either urine or menstrua. It can therefore be used, for example, in the manufacture of infant diapers, and in incontinence products for adults, in addition to the incontinence product specifically identified above.

An example of a sanitary article, typically for the absorption of menstrual fluids is disclosed in WO 94/06386.

A condition of light incontinence exists in many women. An absorbent pad, or other article, for use by those with this condition should desirably

(a) Be thin, and with a shape fitting well into the underpants, to provide discretion when worn under normal clothing.
(b) Be absorbent enough to handle large quantities of urine.
(c) Absorb rapidly enough to accept the surge of urine (gush handling) that can occur from women who have this condition, and maintain this capability even through multiple gushes.

Research indicates that 10-20% of the female population suffer from light involuntary urine losses. The magnitude of the problem varies from losing just a few dribbles in special situations (coughing, sneezing, during sports) to a more serious, permanent problem (after menopause or in conjunction with gynaecological operations). The product selected by such women, and the usage frequency, depends on the seriousness of the problem: pantiliner usage with 1-2 changes per day for occasional urine losses moving to a higher change frequency (2-8 pantiliners/day) for higher loadings and/or more frequent bladder weakness. For those at the upper end of the problem range, pantiliners are not sufficiently absorbent, besides being prone to bunching and to disintegration during use, and such women use 2-3 catamenial pads per day.

Existing products for light incontinence are similar to oversized thick catamenial pads. Most are very thick, about 15mm thick, and this does not provide the degree of discretion the user desires. Furthermore, these products have absorbent cores that typically collapse when wetted, thus making them deficient in fluid absorption rate for subsequent loadings.

For the lightest conditions of light incontinence, many women use standard pantiliners. These products provide the desired level of discretion under clothing; however, they are totally inadequate in absorbency. Part of this deficiency is in absorbent capacity, but more important is the deficiency in absorbent rate.

One object of the present invention is to provide an absorbent article for dealing with light and moderate incontinence, which is discrete, has the absorbent capacity required, and has the necessary gush handling ability.

Our International Patent Application No. PCT/EP94/01814 provides, in one aspect thereof, an article for absorbing fluid, which comprises a fluid-storage region and a fluid-receiving region adapted to release fluid to the fluid-storage region, the fluid-receiving region being formed of a dry laid, for example an airlaid, web of staple fibers, the web having a bulkiness, as measured under a pressure of 2kPa, of at least 15 cm$^3$/g, preferably at least 20 cm$^3$/g. The article described there preferably further comprises a water-permeable topsheet in face-to-face relationship with the said fluid-receiving sheet, on the opposite side thereof to the fluid-storage sheet, and a water-impermeable backsheet in face-to-face relationship with the fluid-storage sheet, on the opposite side thereof to the fluid-receiving sheet. The topsheet and backsheet are preferably sealed to one another, and the article shaped to form a pad suitable for incontinent females.

By "staple fibers" we mean fibers which are not continuous, and which may be synthetic fibers, natural fibers, or a mixture of synthetic and natural fibers.

It is believed that the high bulkiness of the fluid-receiving region is such that the fluid is free to flow with very little impedance by the fibers defining the region. This is in contrast to the approach adopted in known products dealing with incontinence, where any fluid-receiving region serves as a wick to transfer fluid received at one part of the region to other parts thereof. However, it is to be understood that this explanation is offered here as a suggestion only, and no categorical assertion is made that it is correct.

The present invention relates to an absorbent article according to the appended claims.

An object of an aspect of the present invention is to provide advantageous developments of the article of the type described in the above International Patent Application.

Despite the presence of fluid-receiving regions on both sides of the fluid-receiving region, the absorbent article can be thin, being as little as 3mm in thickness, or even less, and provided it has the correct contours to fit well into underwear can be highly discreet. The key is that the article swells only when heavily wetted. This is in contrast to existing products on the market, which if they provide anything approaching an acceptable level of absorbence, are bulky even when dry.

The presence of a second fluid-receiving region, on the opposite side of the fluid-storage region (hereinafter referred to as "the core") to the first fluid-receiving region, increases the temporary storage capacity of the article, enabling a greater quantity of urine or other body liquid to be held until it can be absorbed by the core. The fact that these fluid-receiving regions exist on both sides of the core helps to ensure that the fluid is fed with maximum efficiency to the core. This is particularly important where the core comprises a polymeric hydrogel material (commonly referred to as an AGM material). Such materials are subject to what is known as gel-blocking, where absorption of liquid by one region of the AGM prevents or hinders fluid reaching the remainder of the AGM. Having fluid-receiving regions on both sides of the AGM-containing core means that fluid is efficiently distributed to, and absorbed by, both sides of the core.

Also, having the two fluid-receiving regions, with the core sandwiched between, means that the core is to some extent cushioned against compressive forces which may be applied to the article, thus reducing the possibility of fluid being squeezed out of it. Furthermore, in so far as such squeezing does take place, the fluid which emerges from the core is taken up substantially immediately by the fluid-receiving regions. The fact that, according to this aspect of the invention, such a region is provided on the side of the core nearer the backsheet, as well as on the other side thereof, is particular advantageous, since to the extent that the fluid emerges on one side of the core rather than the other it will be on the lower side, i.e. the backsheet side, as a result of the effect of gravity.

In the accompanying drawings:

Figure 1 is a plan view of a first embodiment of an absorbent article according to the present invention;
Figure 2 is a diagrammatic cross-section, on a larger scale, through the crotch region of the article shown in Figure 1; and
Figure 3 is a view similar to Figure 2, but showing a second embodiment of the invention.

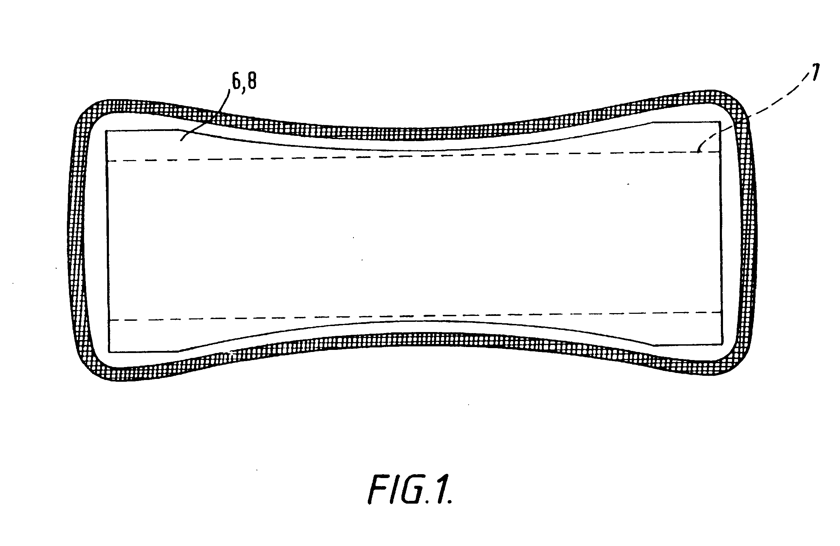

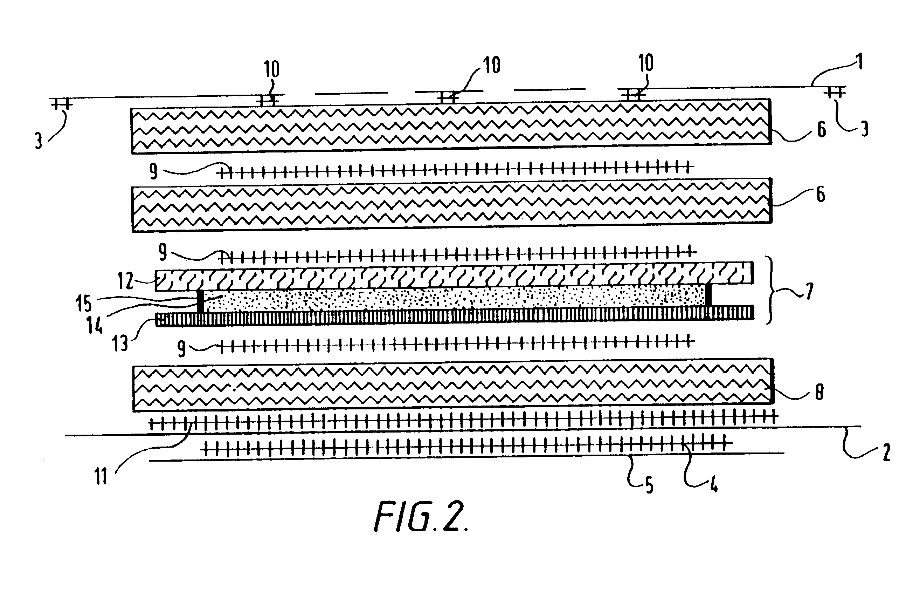

The embodiment of Figures 1 and 2 comprises a liquid permeable topsheet 1 and a liquid impermeable backsheet 2 sealed to one another along a peripheral region 3 thereof by, for example, thermal bonding. The backsheet 2 has a layer 4 of hot-melt adhesive coated thereon, to which is attached a release sheet 5 which is removed by the user before use. Within the enclosure defined by the topsheet 1 and backsheet 2 there are provided an absorbent storage core 7, two secondary topsheets 6 located between the core 7 and the topsheet 1, and a secondary bottom sheet 8 located between the core 7 and the backsheet 2. The sheets 6, core 7 and sheet 8 are attached to one another by layers 9 of cold glue. The layers 9 are discontinuous, so that liquid can pass therethrough. Depending on the capacity required, it may be sufficient to have only one secondary topsheet 6.

The layers 9 are preferably of the type disclosed in our copending International Patent Application No. PCT/EP94/01576 filed on 16th May 1994 and entitled "Adhesive composition". The International Application describes a water-based adhesive composition comprising a blend of adhesive polymers in an aqueous system, characterised in that the blend of adhesive polymers is:

20-60% by weight of an acrylic polymer having a polarity balance expressed as water absorption according to DIN 53495 of 3 to 20%; and correspondingly
40-80% by weight of a compatible tackifying resin having a degree of hydrophobicity measured as the contact angle between a dried film of the resin and a drop of distilled water of not less than 60°;
the percentages being based on the total of acrylic polymer plus tackifying resin expressed as dry solids.

The upper one of the sheets 6 is attached to the topsheet 1 by a plurality of narrow stripes 10 of hot-melt adhesive. The secondary bottom sheet 8 is attached to the backsheet 2 by a layer 11 of hot melt adhesive.

It can be seen that the core 7 consists of three layers, namely upper and lower layers 12 and 13 each of a cellulose-based material, and a middle layer 14 of a water-insoluble hydrogel material (AGM). At the edges of the layer 14 are lines of adhesive 15 which serve to confine the AGM material and prevent liquid therein leaking out.

The absorbent article will now be described in more detail with reference to the four layers thereof.

### 1. Topsheet:

The top layer (user side) is a topsheet that must be comfortable to the touch, provide a dry feeling over an absorbent core filled with liquid, and pass fluid rapidly into the interior of the core. It is liquid permeable in the central longitudinal zone and is liquid impermeable at least in the two lateral zones in order to handle totally the urine during the gush, while avoiding lateral leakage. The width of the liquid impermeable area is such that even when the article is wet to its maximum extent, and is correspondingly swollen, the fluid permeable area of the sheet is not in communication with the lateral edges of the fluid-receiving secondary sheet. This avoid lateral leakage from the core. The liquid impermeable area may extend completely around the liquid permeable area.

This element can be of a variety of known materials, for example: a) a formed-film topsheet as described in U.S. Patent 3929135, or any of European Patent specifications Nos. EP-A-0018020, EP-A-0018684 and EP-A-0059506 (b) a partially perforated fiber/film composite described in EP-A-207904, the perforated area thereof providing a liquid permeable area, and the unperforated area thereof providing a liquid impermeable area, or (c) a nonwoven film produced by the spunbonding or by a carded, thermal-bonded process, or a sheet produced by various other processes currently practised.

2. <u>Secondary topsheets and secondary bottom sheet:</u>

Each of these elements has the characteristics of accepting a high rate of fluid intake, serving as a temporary reservoir for the fluid, and then draining substantially completely into the storage core (or, in the case of the upper secondary topsheet, into the lower secondary topsheet) in order to remain empty for subsequent fluid loadings. In addition, these elements must resist collapse when wet so that they maintain their performance through multiple loadings. The elements must do all these things while also remaining extremely thin. An airlaid web of synthetic fibres can be used for this purpose, the fibres being of a material which is inherently hydrophobic but has been rendered hydrophilic.
The use of fibers which are of a material which is inherently hydrophobic, but is rendered permanently hydrophilic means that the web allows fluid to enter it but also releases the fluid readily to the core, an effect in which the high bulkiness also plays a significant role.
Each of the elements preferably has the same bulkiness, and is preferably made of the same fibers, though the elements may have different thicknesses, and hence different basis weights.
The secondary sheet preferably has the following characteristics:

(a) A thickness of from 1 to 10 mm, more preferably from 1.5 to 6 mm, still more preferably from 1.7 to 4.5 mm, and even more preferably from 2 to 4mm, the thickness being measured with the sheet under a pressure of 2kPa.
(b) A basis weight of from 25 to 300 $g/m^2$, more preferably from in excess of 40 up to 40 to 200 $g/m^2$, still more preferably from 42 or 43 to 200 $g/m^2$, and yet more preferably from 50 to 180 $g/m^2$. Typically, it may be up to 150 $cm^3/g$. For example, basis weights of 45, 60, 80 and 120 $g/m^2$ have been used and found to be satisfactory.
(c) As already mentioned, the sheet has a bulkiness of at least 20 $cm^3/g$, when the sheet is under a pressure of 2kPa. More preferably, the bulkiness is from 20 to 65 $cm^3/g$, still more preferably from 20 to 60 $cm^3/g$, and yet more preferably from 25 to 55 $cm^3/g$. It may advantageously be from 30 to 50 $cm^3/g$. Desirably, the minimum value for the bulkiness is 32, 33, 34 or 35 $cm^3/g$.
(d) The ability to discharge to the storage core at least 95%, and more preferably at least 99% of the fluid which it receives in a loading.
(e) A wet collapse at 2.7kPa of not more than 45%, and more preferably not more than 40%.
(f) A wet resilience at 0.1 kPa of not more than 40%, more preferably not more than 30%, and still more preferably not more than 25%.
(g) A wetting time of not more than 5 seconds, and preferably not more than 2 seconds.
(h) It is formed of fibres having a diameter of not more than 40 $\mu$m, preferably not more than 20 $\mu$m, and still more preferably from 15-20$\mu$m, and a length of not more than 20 mm, preferably not more than 12 mm, and most preferably about 6 mm.

3. <u>Storage Core</u>

The third layer is a thin, high-capacity absorbent core. While thin when dry, this element of the structure preferably expands when wetted to provide a high, tenacious fluid-holding ability, and it must avoid collapse when wet. The storage core is itself preferably formed of a plurality of layers. For example, a three layer structure may be used in which the outer layers are of a cellulose tissue material (and may be the same as, or different from, one another). The middle layer is of a water-insoluble hydrogel (AGM), which is a polymeric material in particulate form, capable of absorbing a large quantity of liquid and retaining it under moderate pressures.
It is important that the secondary topsheets and secondary bottom sheet (hereinafter referred to generically as "secondary sheets") and the storage core work together. In particular, given the form of secondary sheet used herein, it is possible in this structure to avoid the typical problem of gel blocking in the storage core, because the secondary sheets provide total distribution of the fluid, and then drain it, directly or indirectly, into the storage core, from both major faces thereof, whenever the storage core is not saturated.
As an alternative to the form of storage core described above, it can be one of a number of thin, high-capacity materials. For example, the storage core can be a sheet of fused AGM particles as described in International Patent Publications Nos. WO91/14733, WO91/14734, WO91/15362 and WO91/15368 or a high capacity foam, as described, for example, in International Patent Publications Nos. WO93/04092, WO93/03699, WO93/04093, WO93/04113 and WO93/04115.

4. Impervious backsheet

The backsheet is impervious to liquids and, thus, prevents fluid which may be expressed from absorbent core from soiling the body or clothing of the user. Suitable materials are well known in the art, including woven and nonwoven fabrics which have been treated to render them liquid repellent. Breathable or vapour pervious, liquid resistant materials, and those materials described in US-A-3,881,489 and US-A-3,989,867 can also be used. Preferred materials are those materials that are fluid and vapour impervious, because they provide additional fluid strikethrough protection. Especially preferred materials include formed thermoplastic films.

Example 1

The topsheet is a partially perforated fiber/film composite coverstock of the type described in EP-A-0207904. It is partially perforated over a rectangular area which runs lengthwise and centrally of the pad and which has a width of 38 mm.

The secondary sheets are all formed using a hydrophilic resin from Dow Chemical called ASPUN (CODE XU 61518.11) which is a polyethylene resin containing a wetting agent, of the type described in US Patent 4578414. Polyethylene itself is inherently hydrophobic. Bicomponent crimped fibers are formed incorporating this wettable resin internally. The fibers comprise a polypropylene portion and a portion which is formed of the wettable resin mixed with LLDPE (linear, low density polyethylene). At least the latter portion has at least part of its surface exposed to the exterior of the fiber. The fibers are thus rendered permanently hydrophilic. The fibers will normally have a spin finishing agent on their surface. This is provided in order to assist in the spinning process, but it has the incidental effect of rendering the fibers temporarily hydrophilic. However, the spin finishing agent is largely washed off by the first gush of fluid. The wetting agent referred to above, by contrast, makes the fibers permanently hydrophilic.

The fibers are cut into staple fibers 6mm in length, and the staple fibers are airlaid to form a resilient web of wholly synthetic, hydrophilic fibers. The fibers have a diameter of about 18 $\mu$m. The process of airlaying includes the step of applying heat or an adhesive to cause those fibers which touch, or almost touch, one another to bond to each other and those points. Preferably, at least the major part of the fibers of the secondary sheet are the bicomponent fibers, and more preferably substantially 100% are, and most preferably 100% are.

The properties of the secondary sheet thus formed, using 100% bicomponent fibers, and using thermal bonding, are given in the following table under the heading Element 2. By way of comparison, the second column gives the properties of a secondary sheet used in an existing product sold for light incontinence by Kimberly Clark Corporation under the name Poise Pads R.A. For the purpose of the comparison test, a secondary sheet was removed from a Poise pad.

|  | Element 2 | Poise Pad R.A. 2nd Sheet |
|---|---|---|
| Basis Weight (g/m$^2$) | 60 | 130 |
| Caliper (mm @ 2 kPa pressure) | 2.3 | 1.6 |
| Bulkiness (cc/g) @ 2 kPa pressure) | 38.3 | 12.3 |
| Dunk capacity (g/g/ @ P=0) | 36.5 | 20.6 |
| Fluid Retention (g/g) | 0.01 | 1.5 |
| Fluid % discharge * | 99.97 | 92.72 |
| Wet Collapse (% loss @ 2.7 kPa) | 37.1 | 48.7 |
| Wet Resilience (% loss @ 0.1 kPa) | 17.7 | 42.5 |
| Wetting Time (sec) | 0.2 | 7.2 |

*Fluid % discharge = (Dunk capacity - Fluid Retention) /Dunk capacity x 100.

The table above demonstrates the superiority of the secondary sheet used in the invention in each of a number of important performance areas.

The high bulkiness demonstrates that it has a high void volume, and has the ability to acquire fluid efficiently.

The wetting time demonstrates the wettability of the web.

The low fluid retention value demonstrates the ability of the secondary sheet to drain the fluid almost completely (give up fluid into the storage core or other secondary sheet beneath it), so that the secondary sheet drains completely and is therefore available for subsequent loadings.

The low wet collapse and low wet resilience values show that neither the capillary forces of fluid inside the structure, nor external pressure loadings, cause a harmful loss of the open void volume required for the structure to perform well.

The dunk capacity describes the property of being filled substantially totally with urine.

The storage core is a three-layer structure laminate, having the following layers:

a) A top layer having a weight of 75g/m$^2$, of dry-formed, thermal-bonded, cellulose tissue with bicomponent polyolefin staple fibers. The latter are polyethylene-polypropylene ES-C fibers from Danaklon A/S, with a denier of 1.7 dtex and a length of 6mm, the fibers consisting of polypropylene with a polyethylene sheath.
b) Middle layer of particulate (100-800 micron) polyacrylate AGM Dow XZ type (200 g/m$^2$);
c) Bottom layer of air-laid, latex-bonded cellulose embossed tissue (55 g/m$^2$) .

Alternatively, the structure may be made according to our Italian Patent Application No. TO 93 A 001028, which has similarities to what is described in International Patent Publication No. WO94/01069, but which incorporates an AGM material in a higher basis weight.

The backsheet is a 25 $\mu$m coextruded polypropylene/polyethylene film.

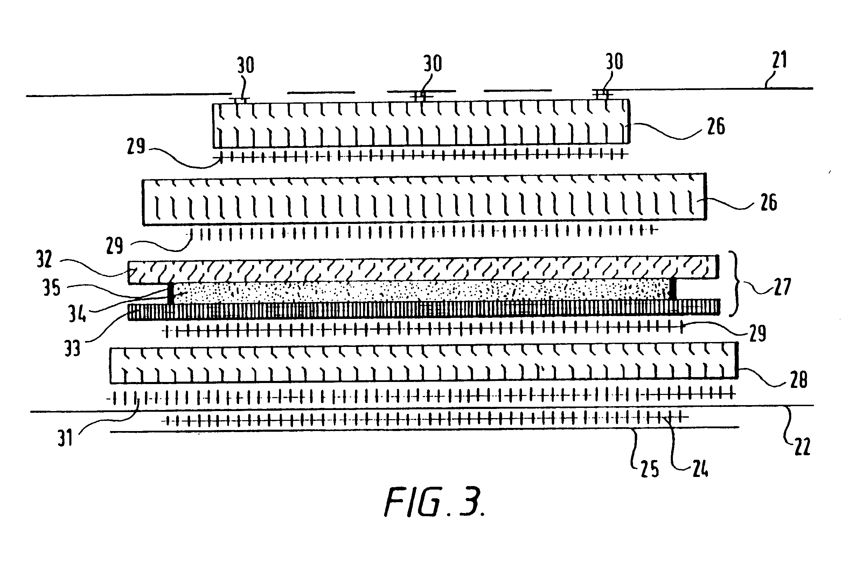

Turning now to Figure 3, this shows an embodiment which incorporates the pyramid feature referred to above. The reference numerals used in Figure 3 corresponds to those used in Figures 1 and 2, but with the addition of 20. It will be seen there is a progressive increase in width as one goes from the upper secondary topsheet 26 to the lower secondary topsheet 26 to the core 27 to the secondary bottom sheet 28. Figure 3 shows the pyramid arrangement as it exists in the crotch region. As in the case of Figures 1 and 2, the secondary topsheets and the secondary bottom sheet are each shaped to have a perimeter which runs approximately parallel to the edge of the article, but the core and both the secondary topsheets have straight side edges. Accordingly, the pyramid effect becomes greater as one moves away from the crotch region, because of the increasing width of the secondary bottom sheet.

It is to be understood that where, as in the case of Figure 3, there are four layers of fluid-receiving or fluid-storing material, there will still be a pyramid if there are two adjacent layers which are of the same width. If there were more than four layers, a pyramid could still be obtained if there were more than two adjacent layers of the same width, or if there was more than the pair of layers of the same width. However, it is also possible for all layers to be greater in width than the layer above it.

In the embodiment shown in Figure 3, the widths of the layers at the crotch are:

| | |
|---|---|
| Upper secondary topsheet (26): | 35mm |
| Lower secondary topsheet (26): | 42mm |
| Core (27): | 50mm |
| Secondary bottom sheet (28): | 54mm |

The maximum width of the secondary bottom sheet is 72mm. It is to be understood that all the dimensions are given by way of example only.

The following sets out the methods used to measure various parameters mentioned above:

Dunk capacity.

This method evaluates the free absorption capacity of the material. A rectangular sample of material 25.4 x 100 mm is put onto the surface of a liquid (synthetic urine, of which the composition is given below) and left on it for one minute. It is then withdrawn by means of a metallic net and left to drip in horizontal position for one minute.

The dunk capacity is obtained as:

```
(Wet weight - dry weight)/dry weight of the sample (g/g).
```

Fluid retention.

The samples obtained from the above test method are rotated in a centrifuge under a g-force of 240 g for ten minutes.

The fluid retention is obtained as:

$$\texttt{(Wet weight - dry weight)/dry weight of the sample (g/g).}$$

Wet collapse.

The samples 38 x 50 mm are made of as many superimposed layers of material as are needed to get an overall basis weight of 500 g/m$^2$. The samples are wetted in the same way as in the dunk capacity test. They are then placed on a perforated plexiglass plate and subjected to three dynamic cycles of compression and decompression (speed of the pressing head 10mm/min, maximum load for each cycle 2.7 kPa). The minimum thickness of the sample under compression is measured.

The wet collapse is:

$$\texttt{(initial thickness - minimum thickness/initial thickness of the sample) x 100 (\%).}$$

Wet resilience.

In the above described test the final thickness of the sample after the last decompression is measured.

The wet resilience is then obtained as:

$$\texttt{(initial thickness - final thickness)/(initial thickness) x 100 (\%).}$$

Wetting time.

In this test, samples of the secondary sheet of the present invention and samples of the second sheet of Poise Pad R.A. having the same volume of about 5 cc are compared. The considered thicknesses correspond to the calipers under pressure (see the values on the table). The samples are placed horizontally onto the surface of water by means of a metallic net. The wetting time is the time needed for each sample to get completely soaked.

Composition of the synthetic urine used in the tests.

The synthetic urine is a solution in distilled water containing the following salts (in weight percent):

Urea 2%, sodium chloride 0.9%, magnesium sulphate (heptahydrate) 0.11%, calcium chloride 0.06%.

**Claims**

1. An article for absorbing body fluid from a wearer, which comprises a fluid-storage region (7) having a first major face on one side thereof and a second major face on the opposite side thereof, and first and second fluid-receiving regions (6, 8) adjacent the said first and second major faces respectively, said article **characterized in that** each fluid-receiving region (6, 8) is adapted to receive fluid and release it to the adjacent major face of the fluid-storage region (7), the fluid-receiving regions (6, 8) each being formed of a material which has a bulkiness, as measured under a pressure of 2 kPa, of at least 20 cm$^3$/g, and a thickness of from 1 to 10 mm, wherein each said fluid-receiving region (6, 8) is in the form of a sheet, and said fluid-storage region (7) is in the form of a further sheet in face-to-face relationship with the fluid-receiving sheets, and the material used for each of the fluid-receiving regions (6, 8) is a dry laid web of staple fibres.

2. An article according to claim 1, wherein the said bulkiness is not more than 65 cm$^3$/g.

3.  An article according to claim 2, wherein the said bulkiness is not more than 60 cm$^3$/g.

4.  An article according to claim 3, wherein the said bulkiness is from 25 to 55 cm$^3$/g.

5.  An article according to claim 4, wherein the said bulkiness is from 30 to 50 cm$^3$/g.

6.  An article according to claim 1, wherein the said thickness is from 1.5 to 6 mm.

7.  An article according to claim 6, wherein the said thickness is from 1.7 to 4.5 mm.

8.  An article according to claim 7, wherein the said thickness is from 2 to 4 mm.

9.  An article according to any preceding claim, wherein the basis weight of the material of the said fluid-receiving region is from 25 to 300 g/m$^2$.

10. An article according to claim 9, wherein the said basis weight is from 40 to 200 g/m$^2$.

11. An article according to claim 10, wherein the said basis weight is from 50 to 180 g/m$^2$.

12. An article according to any preceding claim, wherein each said fluid-receiving region (6, 8) is adapted to release to the fluid-storage region (7) substantially all the fluid which it receives.

13. An article according to claim 12, wherein each said fluid-receiving region (6, 8) is adapted to release to the fluid-storage region (7) at least 95% of the fluid which it receives.

14. An article according to claim 13, wherein each said fluid-receiving region (6, 8) is adapted to release to the fluid-storage region (7) at least 99% of the fluid which it receives.

15. An article according to any preceding claim, wherein the material of each said fluid-receiving region (6,8) has a wet collapse value of not more than 45%, as measured by the wet collapse test method described herein.

16. An article according to claim 15, wherein the said wet collapse value is not more than 40%.

17. An article according to any preceding claim, wherein the material of each said fluid-receiving region (6, 8) has a wet resilience value of not more than 40%, as measured by the wet resilience test method described herein.

18. An article according to claim 17, wherein the said wet resilience value is not more than 30%.

19. An article according to claim 18, wherein the said wet resilience value is not more than 25%.

20. An article according to any preceding claim, wherein the material of each said fluid-receiving region (6, 8) has a wetting time of not more than 5 seconds, as measured by the wetting time test method described herein.

21. An article according to claim 20, wherein the said wetting time is not more than 2 seconds.

22. An article according to any preceding claim, wherein the fibers of each said fluid-receiving region (6, 8) comprises fibres which are of a synthetic plastics material.

23. An article according to claim 22, wherein the material of the fluid-receiving region (6, 8) is hydrophilic.

24. An article according to claim 23, wherein the fibers of each said fluid-receiving region (6, 8) are inherently hydrophobic, but are rendered hydrophilic by the incorporation therein of a wetting agent.

25. An article according to claim 24, wherein at least the major part of the fibers of each said fluid-receiving region (6,8) are bicomponent fibers comprising a polypropylene portion, and a portion which has a surface exposed to the exterior of the fiber and which is formed of a polyethylene resin with the said wetting agent incorporated therein.

26. An article according to claim 25, wherein at least substantially 100% of the fibers of the fluid-receiving region (6,8) are the said bicomponent fibers.

27. An article according to claim 26, wherein 100% of the fibers of each said fluid-receiving region (6, 8) are the said bicomponent fibers.

28. An article according to any preceding claim, wherein the material of each said fluid-receiving region (6, 8) is formed using fibers having a diameter not more than 40 $\mu$m.

29. An article according to claim 28, wherein the said diameter is from 15 to 20 $\mu$m.

30. An article according to any preceding claim, wherein the said fluid-storage region (7) comprises an absorbent hydrogel material.

31. An article according to claim 30, wherein the said fluid-storage region (7) further comprises a cellulose material.

32. An article according to any one of claims 1 to 29, wherein the said fluid-storage region (7) comprises a laminate having outer layers (12, 13) of cellulose-containing material and an intermediate layer (14) of absorbent hydrogel material.

33. An article according to any one of claims 1 to 29, wherein the said fluid-storage region (7) comprises a laminate having outer layers and a central layer of cellulose-containing material, and two further layers of absorbent hydrogel material respectively between the central layer and the two outer layers.

34. An article according to any one of claims 30, 32 and 33, wherein the said absorbent hydrogel material is in particulate form.

35. An article according to claim 1, wherein the said fluid-receiving sheets and the said fluid-storage sheet are secured to one another by an adhesive (9).

36. An article according to claim 1 or 35, further comprising a fluid-permeable topsheet (1) in face-to-face relationship with the said first fluid-receiving sheet, on the opposite side thereof to the fluid-storage sheet, and a fluid-impermeable backsheet (2) in face-to-face relationship with the said second fluid-receiving sheet.

37. An article according to any of claims 1 to 36, wherein at least one of the said fluid-receiving regions (6, 8) is constituted by a plurality of sheet members arranged face to face.

38. An article according to claim 37, wherein said at least one fluid-receiving region (6, 8) is constituted by two sheet members.

39. An article according to any one of claims 36 to 38, wherein the topsheet (1) and backsheet (2) are sealed to one another around the periphery thereof.

40. An article according to any one of claims 1 to 39, which is elongate and has a major axis running along its length and a minor axis running along its width, wherein the sheet nearest that face thereof which is intended to be adjacent a wearer's body has a width less than the sheet adjacent that opposite face which is intended to face away from the wearer's body, and wherein each sheet is at least as wide as the adjacent sheet, if any, nearer the wearer's body and is not wider than the adjacent sheet, if any further away from the wearer's body.

41. An article according to any preceding claim, wherein the material used for each of the fluid-receiving regions (6, 8) has the same bulkiness.

42. An article according to any preceding claim, wherein the material used for each of the fluid-receiving regions (6, 8) is formed of the same fibers.

43. An article according to any preceding claim, shaped to form a pad suitable for incontinent females.

**EP 0 788 335 B1**

**Patentansprüche**

1. Artikel zum Absorbieren von Körperflüssigkeit von einem Träger, umfassend einen Flüssigkeitsspeicherbereich (7) mit einer ersten Hauptfläche auf einer Seite davon und einer zweiten Hauptfläche auf der gegenüberliegenden Seite davon sowie einem ersten und zweiten Flüssigkeitsaufnahmebereich (6, 8) angrenzend an die erste bzw. zweite Hauptfläche,
   wobei der Artikel **dadurch gekennzeichnet ist, dass** jeder Flüssigkeitsaufnahmebereich (6, 8) so ausgelegt ist, dass er Flüssigkeit aufnimmt und an die angrenzende Hauptfläche des Flüssigkeitsspeicherbereichs (7) abgibt, wobei die Flüssigkeitsaufnahmebereiche (6, 8) jeweils aus einem Material gebildet sind, das eine Bauschigkeit, unter einem Druck von 2 kPa gemessen, von mindestens 20 cm$^3$/g und eine Dicke von 1 bis 10 mm aufweist, wobei jeder Flüssigkeitsaufnahmebereich (6, 8) in der Form einer Lage vorliegt und der Flüssigkeitsspeicherbereich (7) in der Form einer weiteren Lage in Fläche-zu-Fläche-Beziehung zu der Flüssigkeitsaufnahmelage vorliegt und das Material, das für jeden der Flüssigkeitsaufnahmebereiche (6, 8) verwendet wird, eine trocken gelegte Bahn von Stapelfasern ist.

2. Artikel nach Anspruch 1, wobei die Bauschigkeit nicht mehr als 65 cm$^3$/g beträgt.

3. Artikel nach Anspruch 2, wobei die Bauschigkeit nicht mehr als 60 cm$^3$/g beträgt.

4. Artikel nach Anspruch 3, wobei die Bauschigkeit von 25 bis 55 cm$^3$/g beträgt.

5. Artikel nach Anspruch 4, wobei die Bauschigkeit von 30 bis 50 cm$^3$/g beträgt.

6. Artikel nach Anspruch 1, wobei die Dicke von 1,5 bis 6 mm beträgt.

7. Artikel nach Anspruch 6, wobei die Dicke von 1,7 bis 4,5 mm beträgt.

8. Artikel nach Anspruch 7, wobei die Dicke von 2 bis 4 mm beträgt.

9. Artikel nach einem der vorstehenden Ansprüche, wobei das Flächengewicht des Materials des Flüssigkeitsaufnahmebereichs von 25 bis 300 g/m$^2$ beträgt.

10. Artikel nach Anspruch 9, wobei das Flächengewicht von 40 bis 200 g/m$^2$ beträgt.

11. Artikel nach Anspruch 10, wobei das Flächengewicht von 50 bis 180 g/m$^2$ beträgt.

12. Artikel nach einem der vorstehenden Ansprüche, wobei jeder Flüssigkeitsaufnahmebereich (6, 8) so ausgelegt ist, dass er im Wesentlichen die gesamte Flüssigkeit, die er aufnimmt, an den Flüssigkeitsspeicherbereich (7) abgibt.

13. Artikel nach Anspruch 12, wobei jeder Flüssigkeitsaufnahmebereich (6, 8) so ausgelegt ist, dass er mindestens 95 % der Flüssigkeit, die er aufnimmt, an den Flüssigkeitsspeicherbereich (7) abgibt.

14. Artikel nach Anspruch 13, wobei jeder Flüssigkeitsaufnahmebereich (6, 8) so ausgelegt ist, dass er mindestens 99 % der Flüssigkeit, die er aufnimmt, an den Flüssigkeitsspeicherbereich (7) abgibt.

15. Artikel nach einem der vorstehenden Ansprüche, wobei das Material jedes Flüssigkeitsaufnahmebereichs (6, 8) einen Nasskollabierwert von nicht mehr als 45 %, wie nach dem hierin beschriebenen Nasskollabierprüfverfahren gemessen, aufweist.

16. Artikel nach Anspruch 15, wobei der Nasskollabierwert nicht mehr als 40 % beträgt.

17. Artikel nach einem der vorstehenden Ansprüche, wobei das Material jedes Flüssigkeitsaufnahmebereichs (6, 8) einen Nasselastizitätswert von nicht mehr als 40 %, wie nach dem hierin beschriebenen Nasselastizitätsprüfverfahren gemessen, aufweist.

18. Artikel nach Anspruch 17, wobei der Nasselastizitätswert nicht mehr als 30 % beträgt.

19. Artikel nach Anspruch 18, wobei der Nasselastizitätswert nicht mehr als 25 % beträgt.

20. Artikel nach einem der vorstehenden Ansprüche, wobei das Material jedes Flüssigkeitsaufnahmebereichs (6, 8) eine Benetzungszeit von nicht mehr als 5 Sekunden, wie nach dem hierin beschriebenen Benetzungszeitprüfverfahren gemessen, aufweist.

21. Artikel nach Anspruch 20, wobei die Benetzungszeit nicht mehr als 2 Sekunden beträgt.

22. Artikel nach einem der vorstehenden Ansprüche, wobei die Fasern jedes Flüssigkeitsaufnahmebereichs (6, 8) Fasern umfassen, die aus einem synthetischen Kunststoffmaterial sind.

23. Artikel nach Anspruch 22, wobei das Material des Flüssigkeitsaufnahmebereichs (6, 8) hydrophil ist.

24. Artikel nach Anspruch 23, wobei die Fasern jedes Flüssigkeitsaufnahmebereichs (6, 8) naturgemäß hydrophob sind, aber durch Einbeziehung eines Benetzungsmittel hydrophil gemacht werden.

25. Artikel nach Anspruch 24, wobei mindestens der Großteil der Fasern jedes Flüssigkeitsaufnahmebereichs (6, 8) Bikomponentenfasern sind, umfassend einen Polypropylenanteil und einen Anteil, bei dem eine Oberfläche zum Äußeren der Faser freiliegt und der aus einem Polyethylenharz mit dem darin eingearbeiteten Benetzungsmittel gebildet ist.

26. Artikel nach Anspruch 25, wobei mindestens im Wesentlichen 100 % der Fasern des Flüssigkeitsaufnahmebereichs (6, 8) die Bikomponentenfasern sind.

27. Artikel nach Anspruch 26, wobei 100 % der Fasern jedes Flüssigkeitsaufnahmebereichs (6, 8) die Bikomponentenfasern sind.

28. Artikel nach einem der vorstehenden Ansprüche, wobei das Material jedes Flüssigkeitsaufnahmebereichs (6, 8) mithilfe von Fasern mit einem Durchmesser nicht mehr als 40 $\mu$m gebildet ist.

29. Artikel nach Anspruch 28, wobei der Durchmesser von 15 bis 20 $\mu$m beträgt.

30. Artikel nach einem der vorstehenden Ansprüche, wobei der Flüssigkeitsspeicherbereich (7) ein absorbierendes Hydrogelmaterial umfasst.

31. Artikel nach Anspruch 30, wobei der Flüssigkeitsspeicherbereich (7) ferner ein Cellulosematerial umfasst.

32. Artikel nach einem der Ansprüche 1 bis 29, wobei der Flüssigkeitsspeicherbereich (7) ein Laminat mit Außenschichten (12, 13) aus cellulosehaltigem Material und einer Zwischenschicht (14) aus absorbierendem Hydrogelmaterial umfasst.

33. Artikel nach einem der Ansprüche 1 bis 29, wobei der Flüssigkeitsspeicherbereich (7) ein Laminat mit Außenschichten und einer Mittelschicht aus cellulosehaltigem Material und zwei weiteren Schichten aus absorbierendem Hydrogelmaterial jeweils zwischen der Mittelschicht und den zwei Außenschichten umfasst.

34. Artikel nach einem der Ansprüche 30, 32 und 33, wobei das absorbierende Hydrogelmaterial in Teilchenform vorliegt.

35. Artikel nach Anspruch 1, wobei die Flüssigkeitsaufnahmelage und die Flüssigkeitsspeicherlage mit einem Klebstoff (9) aneinander befestigt sind.

36. Artikel nach Anspruch 1 oder 35, ferner umfassend eine flüssigkeitsdurchlässige Oberschicht (1) in Fläche-zu-Fläche-Beziehung zu der ersten Flüssigkeitsaufnahmelage, auf der gegenüberliegenden Seite davon zu der Flüssigkeitsspeicherlage, und eine flüssigkeitsundurchlässige Unterschicht (2) in Fläche-zu-Fläche-Beziehung zu der zweiten Flüssigkeitsaufnahmelage.

37. Artikel nach einem der Ansprüche 1 bis 36, wobei mindestens einer der Flüssigkeitsaufnahmebereiche (6, 8) aus mehreren Lagenelementen, die flächig zueinander angeordnet sind, besteht.

38. Artikel nach Anspruch 37, wobei der mindestens eine Flüssigkeitsaufnahmebereich (6, 8) aus zwei Lagenelementen besteht.

**39.** Artikel nach einem der Ansprüche 36 bis 38, wobei die Oberschicht (1) und die Unterschicht (2) an ihrem Umfang miteinander verbunden sind.

**40.** Artikel nach einem der Ansprüche 1 bis 39, der länglich ist und eine Hauptachse, die entlang seiner Länge verläuft, und eine Nebenachse, die entlang seiner Breite verläuft, hat, wobei die Lage, die am nächsten an der Fläche davon ist, die benachbart zum Körper eines Trägers angeordnet sein soll, eine Breite hat, die geringer ist als die der Lage, die benachbart zu der gegenüberliegenden Fläche, die von dem Körper des Trägers abgewandt ist, angeordnet ist, und wobei jede Lage mindestens so breit ist wie die benachbarte Lage, falls vorhanden, die näher am Körper des Trägers ist, und nicht breiter ist als die benachbarte Lage, falls vorhanden, die weiter vom Körper des Trägers entfernt ist.

**41.** Artikel nach einem der vorstehenden Ansprüche, wobei das Material, das jeweils für jeden der Flüssigkeitsaufnahmebereiche (6, 8) verwendet wird, die gleiche Bauschigkeit aufweist.

**42.** Artikel nach einem der vorstehenden Ansprüche, wobei das Material, das jeweils für jeden der Flüssigkeitsaufnahmebereiche (6, 8) verwendet wird, aus den gleichen Fasern besteht.

**43.** Artikel nach einem der vorstehenden Ansprüche, der so geformt ist, dass er eine Binde, die für inkontinente Frauen geeignet ist, bildet.

## Revendications

**1.** Article pour absorber un fluide corporel d'un porteur, qui comprend une région de stockage de fluide (7) ayant une première face principale sur un de ses côtés et une deuxième face principale sur son côté opposé, et des première et deuxième régions de réception de fluide (6, 8) adjacentes auxdites première et deuxième faces principales, respectivement,
ledit article **caractérisé en ce que** chaque région de réception de fluide (6, 8) est adaptée pour recevoir un fluide et le libérer à la face principale adjacente de la région de stockage de fluide (7), les régions de réception de fluide (6, 8) étant chacune formée d'un matériau qui a un encombrement, tel que mesuré sous une pression de 2 kPa, d'au moins 20 cm$^3$/g, et une épaisseur allant de 1 à 10 mm,
dans lequel chaque région de réception de fluide (6, 8) est sous la forme d'une feuille, et ladite région de stockage de fluide (7) est sous la forme d'une autre feuille en relation face à face avec la feuille de réception de fluide, et le matériau utilisé pour chacune des régions de réception de fluide (6, 8) est une feuille par voie sèche de fibres discontinues.

**2.** Article selon la revendication 1, dans lequel ledit encombrement n'est pas plus de 65 cm$^3$/g.

**3.** Article selon la revendication 2, dans lequel ledit encombrement n'est pas plus de 60 cm$^3$/g.

**4.** Article selon la revendication 3, dans lequel ledit encombrement va de 25 à 55 cm$^3$/g.

**5.** Article selon la revendication 4, dans lequel ledit encombrement va de 30 à 50 cm$^3$/g.

**6.** Article selon la revendication 1, dans lequel ladite épaisseur va de 1,5 à 6 mm.

**7.** Article selon la revendication 6, dans lequel ladite épaisseur va de 1,7 à 4,5 mm.

**8.** Article selon la revendication 7, dans lequel ladite épaisseur va de 2 à 4 mm.

**9.** Article selon l'une quelconque des revendications précédentes, dans lequel la masse surfacique du matériau de ladite région de réception de fluide va de 25 à 300 g/m$^2$.

**10.** Article selon la revendication 9, dans lequel ladite masse surfacique va de 40 à 200 g/m$^2$.

**11.** Article selon la revendication 10, dans lequel ladite masse surfacique va de 50 à 180 g/m$^2$.

**12.** Article selon l'une quelconque des revendications précédentes, dans lequel chaque dite région de réception de fluide (6, 8) est adaptée pour libérer à la région de stockage de fluide (7) essentiellement tout le fluide qu'elle reçoit.

13. Article selon la revendication 12, dans lequel chaque dite région de réception de fluide (6, 8) est adaptée pour libérer à la région de stockage de fluide (7) au moins 95 % du fluide qu'elle reçoit.

14. Article selon la revendication 13, dans lequel chaque dite région de réception de fluide (6, 8) est adaptée pour libérer à la région de stockage de fluide (7) au moins 99 % du fluide qu'elle reçoit.

15. Article selon l'une quelconque des revendications précédentes, dans lequel le matériau de chaque dite région de réception de fluide (6, 8) a une valeur d'affaissement humide de pas plus de 45 %, telle que mesurée par le procédé de test d'affaissement humide décrit ici.

16. Article selon la revendication 15, dans lequel ladite valeur d'affaissement humide n'est pas plus de 40 %.

17. Article selon l'une quelconque des revendications précédentes, dans lequel le matériau de chaque dite région de réception de fluide (6, 8) a une valeur de résilience humide de pas plus de 40 %, telle que mesurée par le procédé de test de résilience humide décrit ici.

18. Article selon la revendication 17, dans lequel ladite valeur de résilience humide n'est pas plus de 30 %.

19. Article selon la revendication 18, dans lequel ladite valeur de résilience humide n'est pas plus de 25 %.

20. Article selon l'une quelconque des revendications précédentes, dans lequel le matériau de chaque dite région de réception de fluide (6, 8) a un temps de mouillage de pas plus de 5 secondes, tel que mesuré par le procédé de test de temps de mouillage décrit ici.

21. Article selon la revendication 20, dans lequel ledit temps de mouillage n'est pas plus de 2 secondes.

22. Article selon l'une quelconque des revendications précédentes, dans lequel les fibres de chaque dite région de réception de fluide (6, 8) comprennent des fibres qui sont d'un matériau en plastique synthétique.

23. Article selon la revendication 22, dans lequel le matériau de la région de réception de fluide (6, 8) est hydrophile.

24. Article selon la revendication 23, dans lequel les fibres de chaque dite région de réception de fluide (6, 8) sont de façon inhérente hydrophobes, mais sont rendues hydrophiles par l'incorporation dedans d'un agent mouillant.

25. Article selon la revendication 24, dans lequel au moins la majeure partie des fibres de chaque dite région de réception de fluide (6, 8) sont des fibres bicomposants comprenant une partie de polypropylène, et une partie qui a une surface exposée à l'extérieur de la fibre et qui est formée d'une résine de polyéthylène avec ledit agent mouillant incorporé dedans.

26. Article selon la revendication 25, dans lequel au moins essentiellement 100 % des fibres de la région de réception de fluide (6, 8) sont lesdites fibres bicomposants.

27. Article selon la revendication 26, dans lequel 100 % des fibres de chaque dite région de réception de fluide (6, 8) sont lesdites fibres bicomposants.

28. Article selon l'une quelconque des revendications précédentes, dans lequel le matériau de chaque dite région de réception de fluide (6, 8) est formé en utilisant des fibres ayant un diamètre de pas plus de 40 $\mu$m.

29. Article selon la revendication 28, dans lequel ledit diamètre va de 15 à 20 $\mu$m.

30. Article selon l'une quelconque des revendications précédentes, dans lequel ladite région de stockage de fluide (7) comprend un matériau hydrogel absorbant.

31. Article selon la revendication 30, dans lequel ladite région de stockage de fluide (7) comprend en outre un matériau à base de cellulose.

**32.** Article selon l'une quelconque des revendications 1 à 29, dans lequel ladite région de stockage de fluide (7) comprend un stratifié ayant des couches externes (12, 13) de matériau contenant de la cellulose et une couche intermédiaire (14) de matériau hydrogel absorbant.

**33.** Article selon l'une quelconque des revendications 1 à 29, dans lequel ladite région de stockage de fluide (7) comprend un stratifié ayant des couches externes et une couche centrale de matériau contenant de la cellulose, et deux couches supplémentaires de matériau hydrogel absorbant respectivement entre la couche centrale et les deux couches externes.

**34.** Article selon l'une quelconque des revendications 30, 32 et 33, dans lequel ledit matériau hydrogel absorbant est sous forme particulaire.

**35.** Article selon la revendication 1, dans lequel lesdites feuilles de réception de fluide et ladite feuille de stockage de fluide sont fixées l'une à l'autre par un adhésif (9).

**36.** Article selon la revendication 1 ou 35, comprenant en outre une feuille de dessus perméable aux fluides (1) en relation face à face avec ladite première feuille de réception de fluide, sur son côté opposé à la feuille de stockage de fluide, et une feuille de fond imperméable aux fluides (2) en relation face à face avec ladite deuxième feuille de réception de fluide.

**37.** Article selon l'une quelconque des revendications 1 à 36, dans lequel au moins une desdites régions de réception de fluide (6, 8) est constituée d'une pluralité d'éléments de feuille arrangés face à face.

**38.** Article selon la revendication 37, dans lequel ladite au moins une région de réception de fluide (6, 8) est constituée de deux éléments de feuille.

**39.** Article selon l'une quelconque des revendications 36 à 38, dans lequel la feuille de dessus (1) et la feuille de fond (2) sont jointes l'une à l'autre autour de leur périphérie.

**40.** Article selon l'une quelconque des revendications 1 à 39, qui est allongé et a un grand axe s'étendant le long de sa longueur et un petit axe s'étendant le long de sa largeur, dans lequel la feuille la plus proche de cette face de celui-ci qui est prévue pour être adjacente au corps d'un porteur a une largeur inférieure à la feuille adjacente à cette face opposée qui est prévue pour faire face à l'écart du corps du porteur, et dans lequel chaque feuille est au moins aussi large que la feuille adjacente, le cas échéant, plus proche du corps du porteur et n'est pas plus large que la feuille adjacente, le cas échéant, davantage à l'écart du corps du porteur.

**41.** Article selon l'une quelconque des revendications précédentes, dans lequel le matériau utilisé pour chacune des régions de stockage de fluide (6, 8) a le même encombrement.

**42.** Article selon l'une quelconque des revendications précédentes, dans lequel le matériau utilisé pour chacune des régions de stockage de fluide (6, 8) est formé des mêmes fibres.

**43.** Article selon l'une quelconque des revendications précédentes, profilé de façon à former un tampon approprié pour des femmes incontinentes.

FIG.1.

FIG.2.

FIG.3.

EP 0 788 335 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9406386 A
- EP 9401814 W
- EP 9401576 W
- US 3929135 A
- EP 0018020 A
- EP 0018684 A
- EP 0059506 A
- EP 207904 A
- WO 9114733 A
- WO 9114734 A
- WO 9115362 A
- WO 9115368 A
- WO 9304092 A
- WO 9303699 A
- WO 9304093 A
- WO 9304113 A
- WO 9304115 A
- US 3881489 A
- US 3989867 A
- EP 0207904 A
- US 4578414 A
- IT TO931028 A
- WO 9401069 A